# EUROPEAN PATENT SPECIFICATION

(11) **EP 3 896 079 B1**
(45) Date of publication and mention of the grant of the patent: **12.08.2026**
(21) Application number: 19904564.2
(22) Date of filing: 27.12.2019
(51) Int. Cl.: C07K 14/54, A61K 38/20, C08G 65/32, A61P 37/02, A61K 47/60, A61K 38/00, A61P 31/04, A61P 31/12, A61P 35/00, C07K 14/55, C07K 14/715, C08G 65/333

(54) **METHOD OF PREPARING PEGYLATED BIOMOLECULES HAVING CONTROLLABLE BINDING SITES**
VERFAHREN ZUR HERSTELLUNG PEGYLIERTER BIOMOLEKÜLE MIT KONTROLLIERBAREN BINDUNGSSTELLEN
PROCÉDÉ DE PRÉPARATION DE BIOMOLÉCULES PEGYLÉES AYANT DES SITES DE LIAISON POUVANT ÊTRE CONTRÔLÉS

(30) Priority: 27.12.2018 CN 201811607603
(43) Date of publication of application: 20.10.2021
(73) Proprietor: Jenkem Technology Co. Ltd. (Tianjin), Tianjin 300457 (CN)
(72) Inventor: WANG, Qingbin, Tianjin 300457 (CN); QI, Jie, Tianjin 300457 (CN); LI, Yu, Tianjin 300457 (CN); ZHAO, Xuan, Tianjin 300457 (CN)
(74) Representative: Murgitroyd & Company
(86) International application number: PCT/CN2019/129052
(87) International publication number: WO 2020/135683

(56) References cited:
- WO-A1-2014/144231
- WO-A1-2016/025385
- WO-A1-98/40409
- WO-A1-99/03887
- WO-A2-2004/091517
- WO-A2-2010/023670
- CN-A- 1 871 254
- CN-A- 101 584 866
- US-A1- 2017 369 547

## Description

### TECHNICAL FIELD

The present invention belongs to the technical field of medicine, and particularly relates to a method of preparing PEGylated biomolecules having controllable binding sites. The references to methods of treatment in the subsequent paragraphs are to be interpreted as references to the pharmaceutical compositions and medicaments of the present invention for use in a method for treatment of the human (or animal) body by therapy.

### BACKGROUND

Interleukin-2 (IL-2) is produced when lymphoproliferation is stimulated by phytohemagglutinin (PHA) and other mitogens. Recently, more and more researches on this factor and its receptor have been carried out, and it has been found that the IL-2 plays an important role in cellular immunity, humoral immunity, and immune regulation. In particular, the role and clinical significance of immunotherapy in tumors have been emphasized by many authors.

IL-2 is produced mostly by CD4+ T helper cells (Th) and a few IL-2 is produced by CD8+ and other immune cells. These cells can secrete IL-2 under the action of IL-1. IL-2 produced by lymphocytes acts on cells with IL-2 receptors on the surface, so that the cells can proliferate to play a biological function, and can act on normal T and B lymphocytes, precursor cells of NK cells, cytotoxic T cells (CTL), precursor cells of lymphokine activated killer cells, etc. Biological functions of IL-2 include: 1. maintaining the long-term survival of activated T cells in vitro and maintaining the biological functional activity of the activated T cells, where under the action of IL-2, some T cells activated by tumor antigens can enhance the function of killing tumor cells and enhance the anti-tumor effect; 2. promoting proliferation and differentiation of B cells and generating antibodies; and 3. stimulating secretion of a granulocyte-macrophage colony stimulating factor, γ-interferon, etc., and promoting expression of these receptors in lymphocytes; maintaining the proliferation of thymus in the presence of a subdose of mitogen; and promoting the expression of the IL-2 receptor, participating in an immune regulation system of a body, and maintaining immune balance and stability.

IL-2 is an earliest immunotherapeutic agent. As early as 1992 and 1998, the U.S. FDA approved the IL-2 drug (Aldesleukin) for advanced renal cancer and malignant melanoma with an effective rate of 15-20%, and nearly 10-15% of patients may survive for over 5 years. However, due to high doses for clinical use, IL-2 has significant side effects, such as severe hypotension and vascular leak syndrome. At that time, patients treated with IL-2 must be hospitalized and observed closely around the clock, because little carelessness may cause risk of life. Therefore, the significant side effects limit the use of IL-2, which most clinicians do not dare to use with ease.

The mechanism of action of IL-2 is as follows: IL-2 is produced primarily by antigen-stimulated CD4+ T cells, and may also be secreted by CD8+ T cells, NK cells and DC cells. High doses of IL-2 can promote the differentiation of CD4+ T cells and the proliferation of CD8+ T cells and NK cells, and increase their lethality to mobilize the body's immune response. In turn, low doses of IL-2 can promote the proliferation of regulatory T cells and inhibit the immune system, so low doses of IL-2 are often used to treat autoimmune diseases. This difference is mainly caused by the different distribution of IL-2 receptors on the surfaces of immune cells. The IL-2 receptor totally has three subunits α, β and γ. The receptor on the surfaces of killer cells such as CD8 positive T cells and NK cells mainly has subunits β and γ (IL-2R βγ). This receptor has a low affinity for IL-2 and cannot be activated until high doses of IL-2. Most antibodies on the surfaces of regulatory T cells are high affinity antibodies (IL-2R αβγ) having three subunits α, β and γ, and low doses of IL-2 may activate them more easily to play an immunosuppressive role.

While IL-2 has been approved for the treatment of renal cell tumor and metastatic melanoma more than 20 years ago, it has been abandoned and barely applied in immunotherapy. The main reasons are as follows: 1. the effective rate is low, and the effective rate of IL-2 alone can only reach about 15-20%; 2. IL-2 has a double-edged function, i.e., low doses of IL-2 promotes the proliferation of regulatory T cells but causes immunosuppression, and how to regulate the double-edged function is still to be solved; 3. the half-life is short, i.e., only a few minutes, and large doses must be administered in order to continuously take effect; and 4. adverse reactions are severe, an over-activated immune system may possibly attack self-organs to cause organ failure, and the dosage window is narrow. In order to improve the efficacy of IL-2 and reduce adverse reactions, scientists have also tried various combined solutions, e.g., IL-2+ interferon, IL-2+ lymphokine activated killer (LAK) cell therapy and IL-2+ chemotherapy, which all have an unideal effect.

In recent years, scientists have found: by adding chemical modifications to normal IL-2 molecules, the effect of IL-2 can be maintained, meanwhile the use concentration is reduced, and the side effects are also reduced. Consequently, NKTR-214 was born. NKTR-214 is not mysterious and is essentially an upgraded version of the old drug IL-2 that was marketed more than 20 years ago, but it has lower side effects and higher specificity (higher ease to activate CD8 rather than Treg cells), and may more efficiently activate the immune system. Specifically, six polyethylene glycols (PEGs) are added to an IL-2 molecule for modification to form an inactive drug. Surprisingly, after injection into tumor patients, these six PEG modifications gradually shed, forming active forms of 2-PEG and 1-PEG.

NKTR-214 is PEGylated IL-2 and the activity of the original drug is very low. Six PEG chains were cleverly ligated to sites where the subunit α of IL-2R αβγ high affinity antibodies can bind in IL-2, making PEGylated IL-2 more likely to bind to IL-2R βγ-type receptors in killer immune cells. As the PEGs are degraded one by one, the activity of the drug is released slowly. NKTR-214 addresses two major pain points of IL-2 in tumor therapy: firstly, the effect of drug slow release is achieved through PEG degradation, so that the tolerance is greatly improved; and secondly, the selectivity to the killer immune cells is improved, so that the expression amount of the immune cells PD-1 in the microenvironment is increased.

The Nektar company announced in Nasdaq on October 7, 2015 that a new drug application for NKTR-214 was filed to the U.S. Food and Drug Administration (FDA). The company has completed a preclinical study of NKTR-214 and plans to initiate phase I and phase II clinical studies of the drug by the end of 2015.

In preclinical studies, the AUC value of NKTR-214 in tumors after single dose administration was nearly 400 times that of Aldesleukin compared with an existing IL-2 therapeutic drug Aldesleukin. In addition, the data provided by the Nektar company indicates that NKTR-214 exhibited long-lasting anti-tumor immunotherapeutic effects in multiple preclinical animal models; and in a tumor infiltrating lymphocyte model, after NKTR-214 treatment, the ratio of CD8 positive T cells to CD4 positive T cells was about 450: 1, showing a strong tumor killing effect. Therefore, NKTR-214 is an immune stimulator anti-cancer drug, has a therapeutic effect similar to that of an antibody, and can stimulate the growth of T cells, so as to realize a tumor killing effect. Preclinical experimental results show that the drug has a long-acting anti-tumor therapeutic effect, and is an extremely promising anti-cancer drug.

However, during actual production, 6 PEGs were added to a normal IL-2 molecule by chemical modification to form NKTR-214, so as to selectively bind IL-2 to IL-2R βγ-type receptors. In the present invention, the applicant provides a method of preparing PEGylated biomolecules having controllable binding sites. The binding sites of the biomolecules are artificially interfered in vitro, and the binding sites are kept exposed and not PEGylated, so that the biomolecules are more targeted in vivo, and the therapeutic effect is more rapid. Specifically, when IL-2 is modified, IL-2 is caused to selectively bind to an IL-2R βγ-type receptor by regulating the IL-2 binding sites and PEGylation only adding 1 or 2 PEGs. WO9840409 A1 discloses processes for conjugating proteins with polyethylene glycol. The disclosed processes provide modified proteins having little or no decrease in their activity and include the steps of protecting one or more sites on the protein, contacting the protected protein with polyethylene glycol under conditions suitable for conjugating the polyethylene glycol to the protein, and deprotecting the protein.

WO2016025385 A1 discloses a novel IL-2 protein with selective agonist activity for Regulatory T cells and with an additional amino acid substitution that enable chemical conjugation with Polyethyene Glycol (PEG) that increase circulating half-life compared to the IL-2 selective agonist alone.

### SUMMARY

An objective of the present invention is to provide a method of preparing PEGylated biomolecules having controllable binding sites, and in particular, the present invention provides a method for preparing PEGylated IL-2 having controllable binding sites. The invention is set out in the appended set of claims.

The objective of the present invention is achieved by the technical solutions as below:
A method of preparing PEGylated IL-2 having controllable binding sites comprising the following steps:
(1) preparing an IL-2α receptor affinity column;
(2) binding IL-2 to an IL-2α receptor on the affinity column;
(3) PEGylating, coupling PEG with the IL-2; and
(4) separating the IL-2 from the IL-2α receptor by gradient elution;
   wherein the PEGylated IL-2 has a following structure: 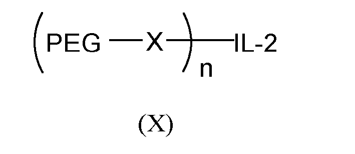
   n is 2;
   X is a linking group between the PEG and IL-2 and is -(CH₂)aCO-;
   a is an integer in the range of 0-10;
   the PEG is a linear monomethoxypolyethylene glycol residue;
   a molecular weight of the PEG is in the range of 10-50 KDa;
wherein the PEGylated IL-2 having controllable binding sites selectively binds to an IL-2 βγ-type receptor.

Preferably, the a is an integer in the range of 0-5 (e.g., 0, 1, 2, 3, 4 or 5). More preferably, the a is an integer in the range of 0-3.

The PEG is a linear monomethoxypolyethylene glycol residue , the molecular weight of the PEG is in the range of 10-50 KDa.

The method of separating the blocker from the biomolecule in the step (3) is gradient elution. Provided is a PEGylated biomolecule having controllable binding sites prepared by the above method.

Provided is a pharmaceutical composition prepared from the PEGylated biomolecules having controllable binding sites prepared by the above method and a pharmaceutically acceptable carrier.

Provided is the pharmaceutical composition prepared from the PEGylated biomolecules having controllable binding sites prepared by the above method and the pharmaceutically acceptable carrier for use in treatment and/or prevention of diseases.

The present invention relates to the biomolecule IL-2, and the blocker an IL-2α receptor (IL-2R α).

The PEGylated IL-2 having controllable binding sites prepared by the above method has a following structure: 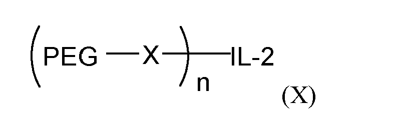
n is 2.
X and PEG have same definitions as preceding definitions. X is a linking group between the PEG and IL-2 and is -(CH₂)aCO-.
a is an integer in the range of 0-10. Preferably, the a is an integer in the range of 0-5 (e.g., 0, 1, 2, 3, 4 or 5). More preferably, the a is an integer in the range of 0-3.

The PEG is a linearmonomethoxypolyethylene glycol (mPEG) residue, the molecular weight of the PEG is in the range of 10-50 KDa.

Provided is a pharmaceutical composition prepared from the PEGylated IL-2 having controllable binding sites prepared by the above method and a pharmaceutically acceptable carrier.

Provided is the pharmaceutical composition prepared from the PEGylated IL-2 having controllable binding sites prepared by the above method and the pharmaceutically acceptable carrier for use in treatment and/or prevention of diseases.

Preferably, the diseases are tumors, autoimmune diseases, viral diseases, or bacterial diseases. The tumor diseases include renal cell carcinoma, melanoma, malignant hemangioendothelioma, cutaneous T cell tumor, ovarian cancer, breast cancer, bladder cancer, lung cancer, glioma, neuroblastoma, liver cancer, hairy cell leukemia, myeloid blast cell leukemia, colon cancer, cancerous pleural effusion, or non-Hodgkin's lymphoma.

The autoimmune diseases include rheumatoid arthritis, systemic lupus erythematosus or sicca syndrome.

Viruses include hepatitis virus, papilloma virus, herpes simplex virus (HSV), human immunodeficiency virus (HIV), Epstein-Barr virus (EBv), coronavirus and influenza virus, more preferably hepatitis virus such as hepatitis B virus (HBV) or hepatitis C virus (HCV). The bacterial diseases include leprosy or tuberculosis.

Unless defined otherwise, all technical and scientific terms as used in the present invention have the same meanings as those usually understood by those skilled in the field of the present invention, for example:
the terms "Interleukin 2", "interleukin 2" and "IL-2" have the same meaning and are used interchangeably herein, may be nature interleukin 2, a recombinant protein (e.g., recombinant human interleukin 2), or a mutant that also has a nature IL-2 function (e.g., "IL-2-C125A/L18M/L19S" as described in the doctoral dissertation "Recombinant Human Interleukin-2 (IL-2) Mutant Cloning and Expression and Purification in Pichia Pastoris System" by LIU Yan), and may also include products obtained by tissue culture, protein synthesis, and cell culture (nature, recombinant cells or mutant) methods. Methods for the extraction and separation of nature, recombinant IL-2 or mutant are well known to those skilled in the art.

As used herein, the term "pharmaceutically acceptable" means physiologically compatible upon administration to a human and does not cause gastrointestinal disorders, such as allergic reactions or similar reactions like dizziness. Additives can be any one of excipients, disintegrants, binders, lubricants, suspending agents, stabilizers, etc. Examples of the excipients include lactose, mannitol, isomalt, microcrystalline cellulose, siliconized microcrystalline cellulose, powdered cellulose, etc. Examples of the disintegrants include low substituted hydroxypropyl cellulose, crospovidone, sodium starch glycolate, croscarmellose sodium, starch, etc. Examples of the binders include hydroxypropyl cellulose, hypromellose, povidone, copovidone, pregelatinized starch, etc. Examples of the lubricants include stearic acid, magnesium stearate, sodium fumaryl stearate, etc. Examples of wetting agents include polyoxyethylene sorbitan fatty acid ester, poloxamer, polyoxyethylene castor oil derivatives, etc. Examples of the suspending agents include hypromellose, hydroxypropyl cellulose, povidone, copovidone, sodium carboxymethylcellulose, methylcellulose, etc. Examples of the stabilizers include citric acid, fumaric acid, succinic acid, etc. In addition, the pharmaceutical composition of the present invention may further include any one of a retarder, a flavoring agent, an emulsifier, a preservative, etc.

The pharmaceutical composition of the present invention may be in the form of tablets (including sugar-coated tablets, film-coated tablets, sublingual tablets, orally disintegrating tablets, buccal tablets, etc.), pills, powder, granules, capsules (including soft capsules, and microcapsules), lozenges, syrups, liquid, emulsions, suspensions, controlled release preparations (e.g., instantaneous release preparations, sustained release preparations, and sustained release microcapsules), aerosol, films (e.g., orally disintegrating films, and oral muco-adhesive films), injections (e.g., subcutaneous injections, intravenous injections, intramuscular injections, and intraperitoneal injections), intravenous dripping agents, transdermal absorption preparations, ointments, lotions, adhesive preparations, suppositories (e.g., rectal suppositories, and vaginal suppositories), pellets, nasal preparations, pulmonary preparations (inhalants), ocular drops, etc., and oral or parenteral preparations (e.g., administration forms of intravenous, intramuscular, subcutaneous, intraorgan, intranasal, intradermal, drip, intracerebral, intrarectal, etc., administration to the vicinity of a tumor and direct administration to a lesion). Preferably, the pharmaceutical composition is an injection.

Preferably, a pharmaceutically acceptable adjuvant of the present invention is preferably a pharmaceutically acceptable injection adjuvant, e.g., an isotonic sterile saline solution (sodium dihydrogen phosphate, disodium hydrogen phosphate, sodium chloride, potassium chloride, calcium chloride, magnesium chloride, etc., or a mixture of the foregoing salts), or a dry, e.g., freeze-dried, composition which is suitably formed into an injectable solute by the addition of sterile water or physiological saline.

The PEGylated IL-2 of the present invention may be abbreviated PEG-IL-2.

### BRIEF DESCRIPTION OF THE DRAWINGS

Fig. 1 is a chromatograph of PEGylation of IL-2 on a receptor affinity column;

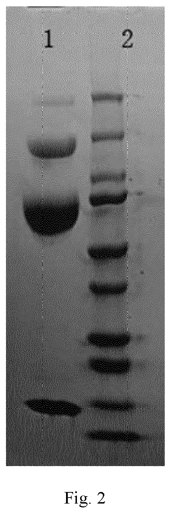

Fig. 2 shows an electrophoresis result of PEG-IL-2, wherein 1 represents the PEG-IL-2 and 2 represents a marker;
Fig. 3 is a chromatograph of gel chromatographic separation of PEG-IL-2; and
Fig. 4 shows an SDS-PAGE result of PEG-IL-2, wherein 1 represents a marker, 2 represents IL-2, 3 represents a peak 2, and 4 represents a peak 1.

### DETAILED DESCRIPTION

The technical solutions in the embodiments of the present invention will be clearly and completely described below. It is obvious that the described embodiments are only a part of embodiments of the present invention, but not all embodiments of the present invention. All the other embodiments obtained by those of ordinary skill in the art based on the embodiments of the present invention without any creative efforts all belong to the protection scope of the present invention.

### Embodiment 1 Preparation of IL-2α receptor affinity column

S1: 0.8 g of CNBr activated sepharose (sepharose CNBr) was weighed, and added into 12 ml of 1 mM HCl, manually and slowly mixed well, and put into a refrigerator at 4°C to be subjected to a reaction for 15 min;
S2: A coupling buffer was prepared: 0.1 M NaHCO₃ (pH=8.3) and 0.5 M NaCl were well mixed, and 12 ml of coupling buffer was taken to wash a filler swollen in the last step;
S3: Four bottles, 4 mg in total, of IL-2α receptor was taken, dissolved in 2 ml of coupling buffer, and added to the filler swollen in the step 2;
S4: Slow rocking-turn was conducted at room temperature for 2 h;
S5: An excess receptor was washed away with 5 column volumes of coupling buffer;
S6: 0.1 M tris(hydroxymethyl)aminomethane (Tris-HCl) (pH=8.0) was added, the mixture was placed at room temperature for 2 h, and the Tris-HCl was poured away;
S7: Washing was conducted with 5 column volumes of 0.1 M acetic acid/sodium acetate buffer (pH=4.0) containing 0.5 M NaCl, and then washing was conducted with 5 column volumes of 0.1 M Tris-HCl buffer (pH=8.0) containing 0.5 M NaCl;
S8: The step 6 and step 7 were repeated for three times;
S9: 0.5 ml of 0.1 M Tris-HCl (pH=8.0) was added; and
S10: A 5ml empty column was filled with sepharose resin, and preserved in the refrigerator at 4°C.

### Embodiment 2 PEGylation of IL-2 on receptor affinity column

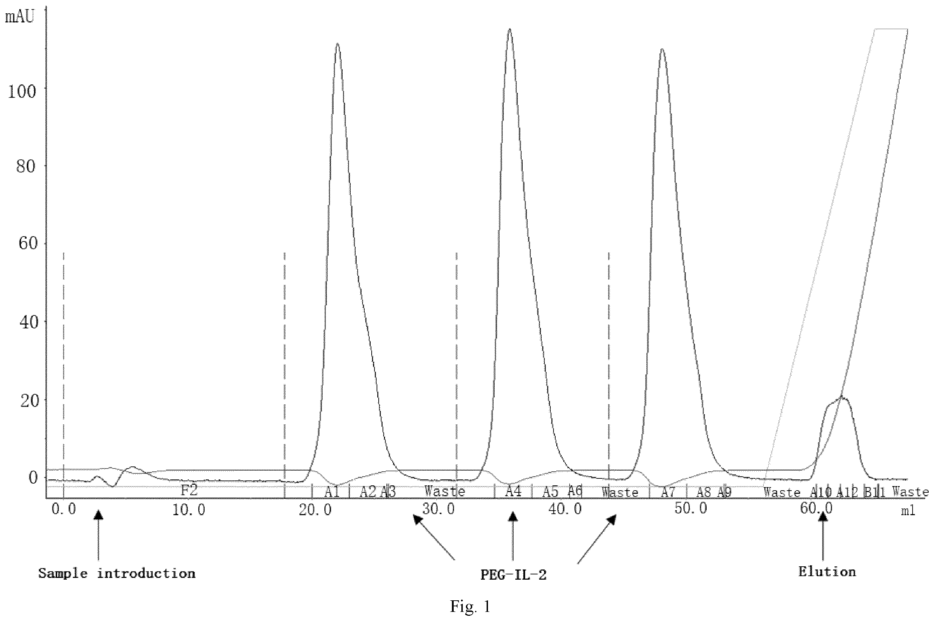

S1: A 5ml receptor affinity column was mounted on a protein purification system (AKTA purifier);
S2: Mobile phases, including a phase A: 5 mM PBS (pH=7.0), and a phase B: a 0.2 M acetic acid solution containing 0.2 M NaCl, were prepared;
S3: A mobile phase flow rate was set to be 0.45 ml/min;
S4: The phase A was balanced to a stable UV curve;
S5: An IL-2 sample was fed through a sampling loop, and an α receptor binding site on IL-2 was bound to an IL-2α receptor;
S6: A proper amount of methoxyl polyethylene glycol-succinimide propionate (M-SPA-20K) with a molecular weight of 20 KDa was taken, and dissolved in 2.5 mM PB (pH=8.0) to a final concentration of 40 mg/ml, 2 ml of M-SPA-20K was sucked, and fed through a sampling loop, and balanced to a stable UV curve;
S7: The step 6 was repeated for three times; and
S8: Gradient elution was conducted, a chromatograph was as shown in Fig. 1, elution peaks were collected, ultrafiltrated, concentrated, and detected by sodium dodecylsulfate-polyacrylamide gel electrophoresis (SDS-PAGE).

### Embodiment 3 gel filtration chromatographic separation of PEG-IL-2

Gel filtration chromatography settings include: a mobile phase: 5 mM PBS, a flow rate: 0.75 ml/min, and a column: superdex 200 increase 10/300 GL.

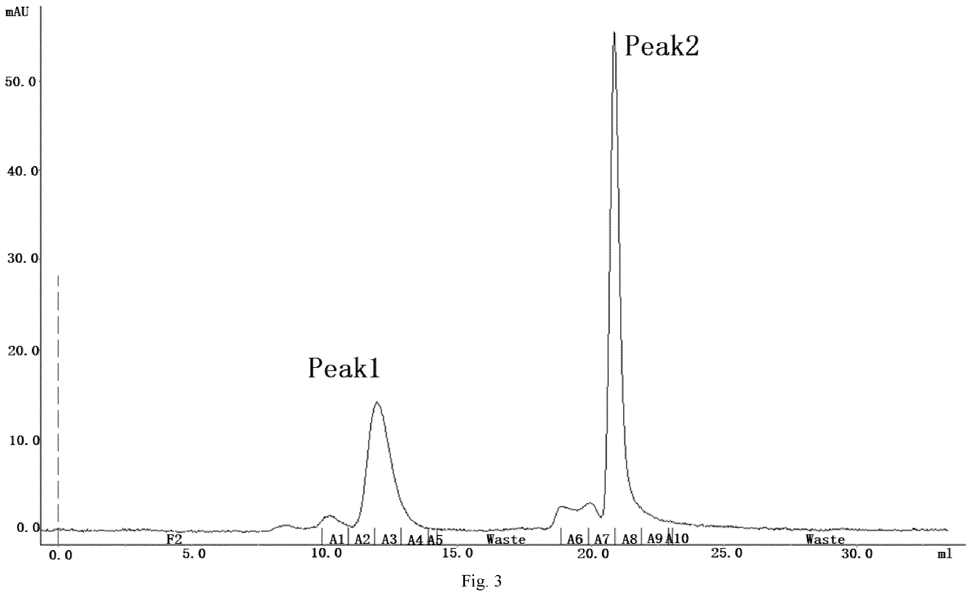

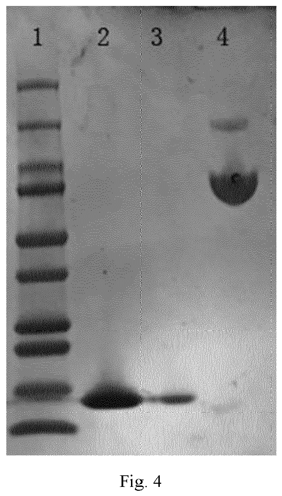

Separation includes: S1: a chromatographic system was balanced to a stable UV curve; S2: elution peaks were collected after sampling; and S3: the collected components were ultrafiltrated, concentrated, and detected by SDS-PAGE, and results were as shown in Fig. 3 and Fig. 4.

Detection results: after confirmation by SDS-PAGE, in Fig. 4, a peak 1 represents PEG-IL-2 and a peak 2 represents IL-2.

### Embodiment 4 Test on receptor affinities

Samples at the peak 1 (PEG-IL-2) and the peak 2 (IL-2) in Fig. 4 were collected, respectively. Test on affinities was carried out by an SPR instrument after concentration. Receptor proteins included an IL-2α receptor and an IL-2β receptor. Test results were as below:

| **Sample** | **PEG-IL-2** | **IL-2** |
|---|---|---|
| **IL-2/IL-2Rα (µM)** | 0.34 | 0.023 |
| **IL-2/IL-2Rβ (µM)** | 31.96 | 2.80 |

Results in the above table show that the PEG-IL-2 prepared by the method of the present invention has a relatively strong binding force to the IL-2β receptor.

## Claims

1. A method of preparing PEGylated IL-2 having controllable binding sites, comprising the following steps:
(1) preparing an IL-2α receptor affinity column;
(2) binding IL-2 to an IL-2α receptor on the affinity column;
(3) PEGylating, coupling PEG with the IL-2; and
(4) separating the IL-2 from the IL-2α receptor by gradient elution;
wherein the PEGylated IL-2 has a following structure: 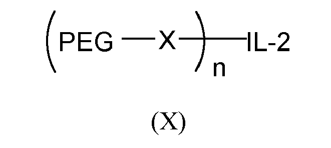
n is 2;
X is a linking group between the PEG and IL-2 and is -(CH₂)aCO-;
a is an integer in the range of 0-10;
the PEG is a linear monomethoxypolyethylene glycol residue;
a molecular weight of the PEG is in the range of 10-50 KDa;
wherein the PEGylated IL-2 having controllable binding sites selectively binds to an IL-2 βγ-type receptor.

2. The preparation method according to claim 1, wherein the a is an integer in the range of 0-5.

3. The preparation method according to claim 2, wherein the a is an integer in the range of 0-3.

4. PEGylated IL-2 having controllable binding sites prepared by the preparation method according to any one of claims 1-3, having a following structure: 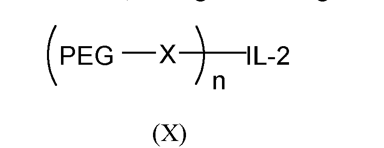
n is 2;
X is a linking group between the PEG and IL-2 and is -(CH₂)aCO-;
a is an integer in the range of 0-10;
the PEG is a linear monomethoxypolyethylene glycol residue;
a molecular weight of the PEG is in the range of 10-50 KDa;
wherein the PEGylated IL-2 having controllable binding sites selectively binds to an IL-2 βγ-type receptor.

5. A pharmaceutical composition prepared from the PEGylated IL-2 having controllable binding sites prepared by the preparation method according to any one of claims 1-3 and a pharmaceutically acceptable carrier.

6. The pharmaceutical composition prepared from the PEGylated IL-2 having controllable binding sites prepared by the preparation method according to any one of claims 1-3 and the pharmaceutically acceptable carrier for use in treatment and/or prevention of diseases.

7. The pharmaceutical composition for the use according to claim 6, wherein the diseases are tumors, autoimmune diseases, viral diseases, or bacterial diseases.

## Patentansprüche

1. Ein Verfahren zum Herstellen von PEGyliertem IL-2 mit kontrollierbaren Bindungsstellen, das die folgenden Schritte beinhaltet:
(1) Herstellen einer IL-2α-Rezeptor-Affinitätssäule;
(2) Binden von IL-2 an einen IL-2α-Rezeptor auf der Affinitätssäule;
(3) PEGylieren, Koppeln von PEG mit dem IL-2; und
(4) Trennen des IL-2 von dem IL-2α-Rezeptor durch Gradientenelution; wobei das PEGylierte IL-2 eine folgende Struktur aufweist: 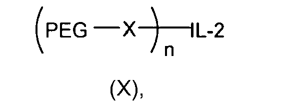
n 2 ist;
X eine Verknüpfungsgruppe zwischen dem PEG und IL-2 ist und -(CH₂)aCO- ist;
a eine ganze Zahl in dem Bereich von 0-10 ist;
das PEG ein linearer Monomethoxypolyethylenglycolrest ist;
ein Molekulargewicht des PEG in dem Bereich von 10-50 kDa liegt;
wobei das PEGylierte IL-2 mit kontrollierbaren Bindungsstellen selektiv an einen IL-2-βγ-Typ-Rezeptor bindet.

2. Herstellungsverfahren gemäß Anspruch 1, wobei das a eine ganze Zahl in dem Bereich von 0-5 ist.

3. Herstellungsverfahren gemäß Anspruch 2, wobei das a eine ganze Zahl in dem Bereich von 0-3 ist.

4. PEGyliertes IL-2 mit kontrollierbaren Bindungsstellen, das durch das Herstellungsverfahren gemäß einem der Ansprüche 1-3 herstellt ist und eine folgende Struktur aufweist: 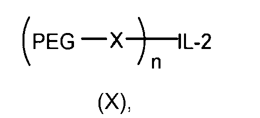
n ist 2;
X ist eine Verknüpfungsgruppe zwischen dem PEG und IL-2 und ist -(CH₂)aCO-;
a ist eine ganze Zahl in dem Bereich von 0-10;
das PEG ist ein linearer Monomethoxypolyethylenglycolrest;
ein Molekulargewicht des PEG liegt in dem Bereich von 10-50 kDa;
wobei das PEGylierte IL-2 mit kontrollierbaren Bindungsstellen selektiv an einen IL-2-βγ-Typ-Rezeptor bindet.

5. Eine pharmazeutische Zusammensetzung, die aus dem durch das Herstellungsverfahren gemäß einem der Ansprüche 1-3 hergestellten PEGylierten IL-2 mit kontrollierbaren Bindungsstellen und einem pharmazeutisch akzeptablen Träger hergestellt ist.

6. Pharmazeutische Zusammensetzung, die aus dem durch das Herstellungsverfahren gemäß einem der Ansprüche 1-3 hergestellten PEGylierten IL-2 mit kontrollierbaren Bindungsstellen und dem pharmazeutisch akzeptablen Träger hergestellt ist, zur Verwendung bei der Behandlung und/oder Prävention von Krankheiten.

7. Pharmazeutische Zusammensetzung zur Verwendung gemäß Anspruch 6, wobei die Krankheiten Tumore, Autoimmunkrankheiten, Viruskrankheiten oder bakterielle Krankheiten sind.

## Revendications

1. Un procédé pour préparer de l'IL-2 PÉGylée ayant des sites de liaison contrôlables, comprenant les étapes suivantes :
(1) préparer une colonne d'affinité pour le récepteur IL-2α ;
(2) lier IL-2 à un récepteur IL-2α sur la colonne d'affinité ;
(3) PÉGyler, en couplant PEG avec l'IL-2 ; et
(4) séparer l'IL-2 du récepteur IL-2α par élution par gradient ;
dans lequel l'IL-2 PÉGylée a une structure suivante : 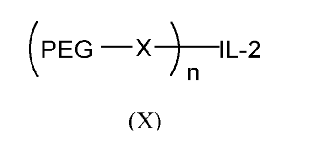
n vaut 2 ;
X est un groupe liant entre le PEG et l'IL-2 et est -(CH₂)aCO- ;
a est un entier dans la plage allant de 0 à 10 ;
le PEG est un résidu de monométhoxypolyéthylène glycol linéaire ;
un poids moléculaire du PEG est dans la plage allant de 10 à 50 kDa ;
dans lequel l'IL-2 PÉGylée ayant des sites de liaison contrôlables se lie sélectivement à un récepteur de type βγ de l'IL-2.

2. Le procédé de préparation selon la revendication 1, dans lequel a est un entier dans la plage allant de 0 à 5.

3. Le procédé de préparation selon la revendication 2, dans lequel a est un entier dans la plage allant de 0 à 3.

4. IL-2 PÉGylée ayant des sites de liaison contrôlables préparée par le procédé de préparation selon l'une quelconque des revendications 1 à 3, ayant une structure suivante : 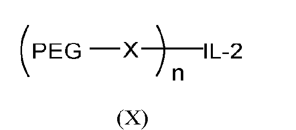
n vaut 2 ;
X est un groupe liant entre le PEG et l'IL-2 et est -(CH₂)aCO- ;
a est un entier dans la plage allant de 0 à 10 ;
le PEG est un résidu de monométhoxypolyéthylène glycol linéaire ;
un poids moléculaire du PEG est dans la plage allant de 10 à 50 kDa ;
dans laquelle l'IL-2 PÉGylée ayant des sites de liaison contrôlables se lie sélectivement à un récepteur de type βγ de l'IL-2.

5. Une composition pharmaceutique préparée à partir de l'IL-2 PÉGylée ayant des sites de liaison contrôlables préparée par le procédé de préparation selon l'une quelconque des revendications 1 à 3 et d'un support pharmaceutiquement acceptable.

6. La composition pharmaceutique préparée à partir de l'IL-2 PÉGylée ayant des sites de liaison contrôlables préparée par le procédé de préparation selon l'une quelconque des revendications 1 à 3 et du support pharmaceutiquement acceptable pour une utilisation dans le traitement et/ou la prévention de maladies.

7. La composition pharmaceutique pour l'utilisation selon la revendication 6, dans laquelle les maladies sont des tumeurs, des maladies auto-immunes, des maladies virales ou des maladies bactériennes.
